(19) Europäisches Patentamt
European Patent Office
Office européen des brevets

(11) Publication number: **0 085 367**
**A1**

(12) # EUROPEAN PATENT APPLICATION

(21) Application number: 83100539.2

(22) Date of filing: 21.01.83

(51) Int. Cl.³: C 07 C 85/06
C 07 D 295/12

(30) Priority: 26.01.82 US 342952

(43) Date of publication of application:
10.08.83 Bulletin 83/32

(84) Designated Contracting States:
DE FR GB IT

(71) Applicant: AIR PRODUCTS AND CHEMICALS, INC.
P.O. Box 538
Allentown, Pennsylvania 18105(US)

(72) Inventor: Bechara, Ibrahim Selim
1159 Naaman's Court
Boothwyn, PA 19061(US)

(72) Inventor: Milligan, Barton
RD 1
Coplay, PA 18037(US)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Amination of hydrogen sulfate esters of hydroxyalkyl tertiary amines.**

(57) Ditertiary amines are formed in high yield by reacting hydrogen sulfate esters of tertiary alkanol amines with excess of secondary amine at temperatures below 100°C. In a preferred embodiment the hydrogen sulfate ester of N-hydroxyethyl morpholine is reacted with dimethylamine.

EP 0 085 367 A1

122-P-US02675

# AMINATION OF HYDROGEN SULFATE ESTERS OF HYDROXYALKYL TERTIARY AMINES

## TECHNICAL FIELD

The present invention relates to the preparation of ditertiary amines and is particularly concerned with methods for selectively synthesizing the desired products at high yields.

## BACKGROUND OF THE PRIOR ART

Ditertiary amines are useful products as curing agents and catalysts for polyurethanes, epoxies and other thermoset plastics. Among the starting materials found attractive on an industrial scale for the preparation of ditertiary amines are hydroxyalkyl tertiary amines. These tertiary amines are usually converted to the ditertiary amine compounds by a gas phase process employing noble metal catalysts and high temperatures. Such processes, unfortunately, result in excessive disproportionation and/or cracking of the starting material and the product, thereby reducing selectivity for production of the desired ditertiary amine and creating other undesirable amine products that must be disposed of. Other potential routes for the production of ditertiary amines as by suggested liquid phase processes, usually involve numerous steps that make the manufacture relatively costly and at times involve the

use of intermediates that are difficult to handle, such as alkyl chlorides, chloroethyl ethers or chloroethyl amines. In addition, certain of these intermediates are hazardous, such as those of the chloroethyl morpholine type, which is among the nitrogen mustards.

An article by E. Cherbuliez et al. published in Helvetica Chimica Acta (Vol. 47, Fasiculus 7 (1964) - No. 234, at pages 2106-2112) describes research on the esterification of amino alcohols with sulfuric acid. The obtained esters provide an intermediate for the synthesis of alkylene imines. Among the reactions described in the publication is neutralization of triethanol amine with sulfuric acid followed by heating the obtained addition salt at 190°C under vacuum, obtaining at first bis (hydroxyethyl) amino ethyl sulfuric ester, which on further heating is reported to be cyclicized to form morpholine N-hydroxy-ethyl hydrogen sulfate.

German Patent No. 744.996 (1944) discloses a general method stated to be applicable for the preparation of certain acyclic and heterocyclic ditertiary amines. By the methods described in the patent, acid sulfate esters of lower hydroxyalkyl amines, or inner salts thereof, are reacted at elevated temperature and superatmospheric pressure with ammonia or with primary or secondary aliphatic amines or with secondary nitrogen ring bases. The reaction of the amine with the amino alkyl sulfuric acid compound is carried out in an autoclave for 12 to 16 hours at temperatures in the range of 160-190°C. Among the examples of the German patent, there is described the synthesis of N-(ß-aminoethyl) piperidine by reaction of piperidine with ß-aminoethyl sulfuric acid in an autoclave for 14 hours at 170°C.

## SUMMARY OF THE INVENTION

It has now been unexpectedly found that high yields of desired tertiary amines can be obtained by reaction of a secondary amine, preferably a short chain dialkyl amine, with the hydrogen sulfate ester of a tertiary alkanol amine, if the reaction is carried out at temperatures below 100°C, particularly at a temperature in the range of 50-80°C and by employing a large excess of the secondary amine. The general reaction scheme is illustrated by the equation below as applied to the amination of the hydrogen sulfate ester of a hydroxyalkyl tertiary amine with a dialkyl amine:

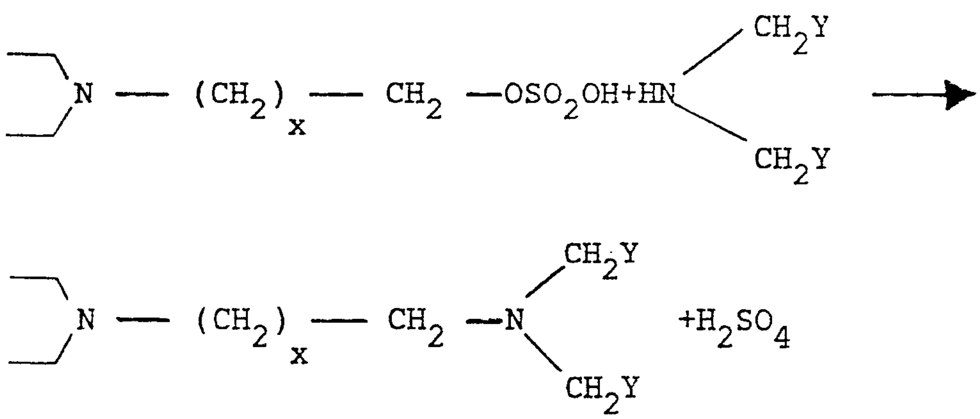

wherein x is 1 to 2 and Y is H or $CH_3$.

Thus, by reaction of the hydrogen sulfate ester of N-hydroxyethyl morpholine with dimethylamine there is obtained the tertiary amine, N-(dimethylaminoethyl) morpholine (DMAEM), a commercial catalyst, one brand of which is known in the trade as "DABCO XDM".

## DETAILED DESCRIPTION OF THE INVENTION

Any of the known methods of the prior art may be employed for forming the intermediate sulfate ester of the hydroxyalkyl tertiary amine. For example, the desired sulfate ester intermediate for production of the ditertiary amine derivative of morpholine can be obtained by the following steps:

1. Reacting morpholine with ethylene oxide at 50-100°C in aqueous solution, obtaining yields in excess of 90+%.
2. Adding concentrated sulfuric acid to the crude N-hydroxyethyl morpholine (HEM). Since removal of water favors the reaction, this may be accomplished with the use of xylene as an azeotrope or by carrying out the esterification under moderate vacuum.

For conversion of the sulfate ester of the hydroxyalkyl tertiary amine to the desired ditertiary compound, it is dissolved in water and excess dialkylamine added to the solution. The mixture is heated for several hours at below 100°C, preferably at a temperature not in excess of 80°C. The desired ditertiary amine product is recovered by addition of sodium hydroxide followed by solvent extraction and atmospheric distillation.

The hydrogen sulfate esters of alkanol amines are unstable in aqueous solution at temperatures above about 100°C. At such higher temperatures these esters will undergo a hydrolysis reaction reverting back to the starting hydroxylamines. It is therefore important to carry out the amination at below 100°C and preferably below 80°C.

When the secondary amine is added in stoichiometric proportion to the hydrogen sulfate ester of the alkanolamine, the reaction does not go to completion since some of the secondary amine is tied up as ammonium sulfate. It is therefore advocated, in accordance with the invention, that a large excess of the secondary amine be used to assure the desired higher yields of the ditertiary amine product. The large excess of secondary amine is also helpful since part of it is needed to free the product from its sulfuric acid salt.

Example 1

In a laboratory scale program, the hydrogen sulfate ester of hydroxyethyl morpholine was reacted in aqueous solution with dimethyl amine, in the proportions of 2 mols dimethylamine per mol of ester. The reaction was carried out at 50°C.

An 80% yield of ditertiary amine was obtained as determined by gas-liquid chromatography (glc).

Table 1 below compares the above yield with that of other runs made under listed conditions outside of those advocated by the present invention.

TABLE 1

| Example | Molar ratio DMA/sulfate ester of HEM | Reaction T °C | % yield ditertiary amine |
|---|---|---|---|
| 1 | 2.0 | 50 | 80 |
| 2 | 3.12 | 125 | 48 |
| 3 | 0.94 | 80 | 50 |
| 4 | 1.08 | 125 | 20 |

It will be seen from the above table that at temperatures above 100°C or in the absence of a sufficient excess of the secondary amine, comparatively low yields of the desired ditertiary amine were obtained.

It will be understood that other secondary amines can be reacted with sulfuric esters of N-hydroxy alkyl morpholine or with such esters of other hydroxyalkyl N-hetero tertiary amines, in the same manner and substantially under the same conditions advocated herein.

Further improvement in yield of desired diteriary amine may be achieved by adding the dry hydrogen sulfate ester to an aqueous solution of the secondary amine, thus minimizing the hydrolysis of the ester.

P14-F

What Is Claimed:

1. The method of producing ditertiary amines which comprises reacting a hydrogen sulfate ester of a tertiary alkanol amine with a stoichiometric excess of a secondary amine at temperatures below 100°C.

2. The method as defined in Claim 1 wherein the reaction is carried out at a temperature in the range of about 50°-80°C.

3. The method as defined in Claim 1 wherein said secondary amine is a short chain dialkylamine.

4. The method as defined in Claim 1 wherein said secondary amine is dimethylamine.

5. The method as defined in Claims 1, 2, 3 or 4 wherein said sulfate ester is that of a tertiary alkanolamine in which the tertiary N of the amino group is linked to carbon in a ring compound arrangement.

6. The method according to Claims 1, 2, 3 or 4 wherein said sulfate ester corresponds to the formula.

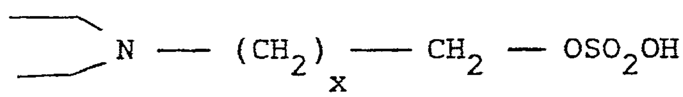

wherein the nitrogen atom of the formula is part of a heterocyclic ring and x is 1 or 2.

7. The method according to claims 1, 2, 3 or 4 in which the sulfate ester is that of N-hydroxyethyl morpholine.

8. The method which comprises reacting the hydrogen sulfate ester of N-hydroxyethyl morpholine with an excess of short chain dialkyl amine at a temperature of no greater than about 80°C

and recovering the ditertiary nitrogen morpholine compound thus formed.

9. The method as defined in Claims 8 wherein said short chain dialkyl amine is dimethylamine.

European Patent Office

**EUROPEAN SEARCH REPORT**

0085367

Application number

EP 83 10 0539

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | FR-A-1 448 364 (ARMOUR) *Abstract* | 1 | C 07 C 85/06<br>C 07 D 295/12 |
| Y | GB-A-2 006 200 (BAYER) *Claims* | 1-5 | |
| Y | US-A-3 843 648 (I.S.BECHARA) *Claims* | 1,6 | |
| D,Y | HELVETICA CHEMICA ACTA, vol. 47, no. 7, 30th October 1964, pages 2106-2112; E.CHERBULIEZ et al.: "Recherches sur la formation et la transformation des esters LVI[1]1) Action de l'acide sulfurique sur les aminoalcools". *Pages 2107-2109* | 1 | |
| | | | TECHNICAL FIELDS SEARCHED (Int. Cl. 3) |
| | | | C 07 C 85/00<br>C 07 D 295/00 |

The present search report has been drawn up for all claims

| Place of search | Date of completion of the search | Examiner |
|---|---|---|
| THE HAGUE | 29-04-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82